# EUROPEAN PATENT APPLICATION

(11) **EP 2 679 277 A1**
(43) Date of publication of application: **01.01.2014**
(21) Application number: 12174160.7
(22) Date of filing: 28.06.2012
(51) Int. Cl.: A61N 5/10

(54) **Apparatus and method for conformal particle radiation therapy of a moving target**

(71) Applicant: Ion Beam Applications, 1348 Louvain-la-Neuve (BE)
(72) Inventor: Jongen, Yves, 1348 Louvain-la-Neuve (BE)
(74) Representative: De Groote, Christophe

(57) **Abstract**

A particle therapy assembly for irradiating a moving target with a particle beam is provided. The assembly comprises breath holding means for holding a patient's breath during the delivery of the particle beam to the target. With the assembly and method of invention, displaying means are provided to the patient to visualize the actual position of the moving target together with a prescribed target position, so that the patient can manage his breath and actively contribute to the target positioning before actuating the breath holding means and starting the delivery of the particle beam.

## Description

### Field of the invention

The invention relates to a particle therapy assembly for irradiating a moving target in a patient with a particle beam. The particle therapy assembly comprises a particle beam generator for generating a particle beam and a scanning device for scanning the target with the particle beam. Such a scanning device comprises one or more scanning magnets for scanning the particle beam over an X-Y scanning plane and scanning control means configured for sequentially scanning the particle beam by moving the beam to the multiple scanning positions situated in the X-Y scanning plane. The scanning positions are defined by taking into account the shape of the target such that when scanning the target dose conformation is obtained. The particle therapy assembly further comprises a couch for positioning the patient in an irradiation treatment position.

The invention also relates to a method for positioning a moving target in a patient.

### Description of prior art

Several types of particle therapy assemblies having a scanning device with scanning magnets for directing and delivering the beam to an area of the target are known. By directing and delivering the beam sequentially to multiple scanning positions an entire target can be irradiated with the particle beam. The number of positions to be irradiated to cover the entire target depends on the target size and the particle beam size. In general, the scanning positions are defined as (x,y) coordinates in an X-Y scanning plane, which is a plane perpendicular to a main beam direction Z, said main beam direction Z being a direction followed by the particle beam at an exit of the scanning device when the scanning magnets are not energized.

Intra-fractional target motions, which are mainly caused by respiration of the patient, require adaptations in the particle therapy assembly in order to satisfy requirements related to dose conformation. Indeed, not taking into account target motion results in a blurring of the dose gradients from target volume to normal (healthy) tissue and, in addition, during target motion the radiological beam path length can change, which could result in under-dosage or over-dosage of the target volume.

The known different types of particle therapy assemblies adapted for irradiating moving targets with scanning beams are further discussed.

A first type of particle therapy assembly for irradiating moving targets is using the so-called beam gating technique. With this technique, based on a signal from a target motion-monitoring device, the beam delivery to the target is controlled such that beam delivery only occurs during specific phases of the breathing cycle. Those assemblies are complex as synchronization is needed between the beam on/off controls and the phases of the breathing cycle. As the beam delivery only takes place during a fraction of the breathing cycle, the irradiation time is also increased.

A second type of particle therapy assembly for irradiating moving targets with a scanning device is using the so-called re-scanning or re-painting technique. With this technique the target is irradiated multiple times with a partial dose in order to smear out the effect of organ motion. A disadvantage of such a technique is that healthy tissue may nevertheless be irradiated here and there with partial doses. Another disadvantage is that the re-paintings are time consuming so that the overall treatment time is increased. A repainting technique is for example described in EP2392383.

A third type of particle therapy assembly for irradiating moving targets is using the beam tracking technique. With this technique, the lateral position of the beam and/or beam energy are adjusted during beam delivery for compensating for organ motion. This type of assembly is also complex and requires for a 4D treatment planning system. For example, in US2011105821, a system is provided to regulate the energy of the beam during the patient's irradiation depending on the detection of a target motion.

### Summary of the invention

It is therefore an object of the invention to provide an improved particle therapy assembly specifically adapted for irradiating moving targets.

These objects and other aspects of the invention are achieved with the assembly and methods as claimed.

To this end, the particle therapy assembly according to the invention comprises:
● an imaging device for acquiring an actual position of the moving target while the patient is in the treatment position, said imaging device comprising a display and displaying controls configured for displaying, in real time, on the display, information indicative of an actual position of the moving target and information indicative of a prescribed target position;
● breath holding means for holding a breath of the patient while the patient is positioned in the treatment position;
● means for actuating the breath holding means to start holding the patient's breath;
● means for starting the irradiation of the target with the particle beam.

By providing a display showing the actual position of the moving target together with the prescribed target position, the breath holding means can be actuated when the target is effectively in the right position (= the prescribed target position) for performing the particle radiation treatment. The breath holding means comprises, for example, an active breath control (ABC) device. With an active breath control device, the airflow of the patient is temporarily blocked by a valve. An actuation button, can, for example, by used for actuating the breath holding means for controlling the valve.

Preferably, the particle assembly according to the invention, comprises means for positioning the display such that the patient can visually observe the display while the patient is in the irradiation treatment position.

More preferably, the display is in a display position which is such that said patient can visually observe the information indicative of the actual position of the moving target and the information indicative of a prescribed target position while the patient is in the irradiation treatment position.

When the patient observes the information indicative of the actual position of the target and the information indicative of the prescribed target position, the patient can, based on this information, manage his breath such that the actual position of the target is within a tolerance of the prescribed target position. At that moment, the breath holding means can be actuated such that the target maintains in the good position for treatment (=prescribed target position). This improves the reproducibility of target positioning.

The particle therapy assembly according to the invention is more preferably characterized in that it comprises :
● computing means for comparing in real time the actual position of the moving target with the prescribed target position; and
● means for providing a signal when the actual position of the moving target matches the prescribed target position within a tolerance.

In this way, the patient receives a feedback while managing his breath for adjusting the position of the moving target. The mentioned tolerance for positioning the target is a parameter that is defined by a medical doctor. Examples of parameter values for said tolerance are 30 mm, or 20 mm, or 10 mm, or 5 mm, or 2 mm. Said tolerance is in general smaller than 50 mm, preferably smaller than 30 mm and more preferably smaller than 20 mm.

In a more preferred embodiment, the particle assembly according to the invention further comprises means for synchronizing the means for starting the irradiation of the target and the means for actuating (80) the breath holding means. In this way, the start of the irradiation can be performed automatically without delay following the actuation of the breath holding means. Hence, the time the patient has to hold his breath can be strictly limited to the time needed to irradiate the target.

The starting of the irradiation is, preferably, performed simultaneously with the actuation of the breath holding means.

The imaging device is, for example, comprising a fluoroscopic imaging device. Other imaging modalities, known in the art, for visualizing the target of the patient can be used as well (e.g. ultrasound, ...). Also indirect imaging devices can be applied such as e.g. the use of detectors measuring motion or expansion of the patient's surface.

Furthermore, computing means are preferably provided for computing an expected irradiation time period. In this way, one knows in advance how long the patient has to hold his breath and the patient can be trained to hold his breath for the specific time period needed to perform the irradiation of the target.

The assembly according to the invention further comprises means for modulating an energy of the particle beam as function of the scanning positions. Preferably, in the assembly according to the invention, the means for modulating the energy are located downstream from the one or more scanning magnets of the beam delivery device. In this way, with a downstream range modulation, for each scanning position, a range modulation can be specified and a corresponding Spread-Out-Bragg Peak created in the target for each scanning position. In this way, there is no need to follow a time consuming sequence of making irradiations layer per layer for which upstream energy changes are needed. In other words, the irradiation can be performed by a single scan of the beam over the different scanning positions. This strongly reduces to overall irradiation period for delivering a prescribed dose to the target.

Hence, the irradiation can be completed within a single breath hold period. This makes the use of an active breath holding device in combination with such a particle beam scanning device an attractive tool for irradiating moving targets.

In a preferred embodiment of a particle therapy assembly according to the invention having downstream energy modulation, the means for modulating the energy comprises an energy filter having a plurality of filtering elements. Each of the plurality of filtering elements is configured for modulating the energy of the particle beam and is associated to one of the scanning positions of the particle beam in the X-Y scanning plane. The energy filter is configured such that when the beam is directed to a scanning position, the associated filtering element is being crossed by the particle beam. The advantage is that such an energy filter can be custom made for each target to be irradiated and hence be specifically adapted to the shape of the target. Target dose conformation is hereby increased.

It is a further object of the invention to provide a method for positioning a moving target of a patient. This is achieved with the methods as claimed.

### Short description of the drawings

These and further aspects of the invention will be explained in greater detail by way of example and with reference to the accompanying drawings in which:
- Fig.1: shows a schematic view of an example of particle therapy assembly;
- Fig.2: shows a schematic view of an example of a particle therapy assembly according to the invention;
- Fig.3: shows a schematic representation of an example of an energy filter;
- Fig.4: shows a schematic representation of multiple examples of filtering elements;

The figures are not drawn to scale. Generally, identical components are denoted by the same reference numerals in the figures.

### Detailed description of preferred embodiments

According to a first aspect of the invention a particle therapy assembly (100)is provided for irradiating moving targets.

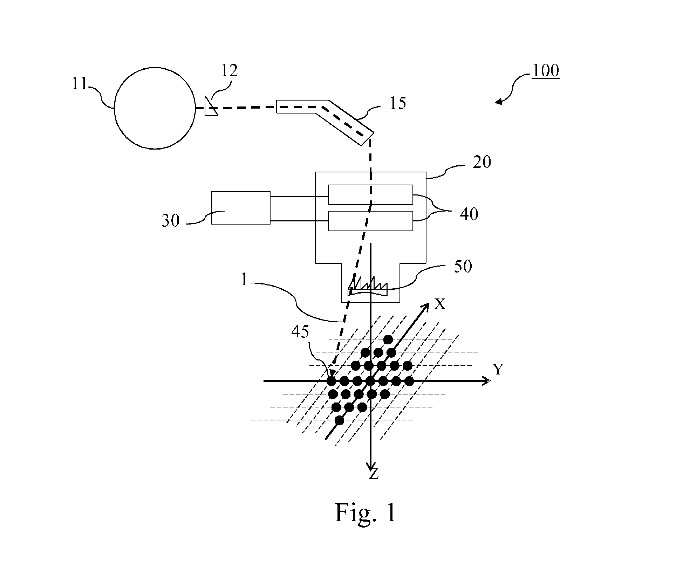

Fig. 1 shows a schematic view of an example of particle therapy assembly (100) according to the invention (not all components are shown on the figure). The assembly comprises a particle beam generator for generating a particle beam (e.g. proton or carbon ion beam). As an example, a particle beam generator comprises a fixed energy cyclotron (11) and an energy degrader (12) for degrading the fixed energy such that a particle beam of a selected energy can be delivered. For example, beams with a proton generator can actually be delivered with an energy varying between 70 MeV and 230 MeV. Another example of a particle beam generator is a synchrotron where the energy of the beam extracted from the synchrotron can be selected. The particle beam having a selected energy is then, using a transport system (15), further transported to a scanning device (20) configured for scanning the target. The scanning device (20) is adapted for delivering the particle beam in an appropriate form to a target volume within a patient. Basically, the scanning device shapes the dose distribution to the target volume to be irradiated. The scanning device (20) comprises one or more scanning magnets (40) for scanning the particle beam. In general, the scanning device (20) comprises two scanning magnets (40) for scanning the particle beam in an X-Y scanning plane, or, alternatively, a single double direction scanning magnet (40)can be used to scan the beam in the two directions X and Y. In Fig. 1, a particle beam (1) is illustrated as a dashed line. The scanning device further comprises scanning control means (30) for controlling the scanning magnets (40). The scanning control means (30) are configured for scanning the particle beam by sequentially moving the particle beam to multiple scanning positions (45) situated in the X-Y scanning plane. This is illustrated in Fig. 1 where, as an example, a number of scanning positions (45) are shown in an X-Y plane. In general, the X-Y plane is perpendicular to a central axis Z of the scanning device (20), this central axis Z is defined as the direction the particles follow if the beam is not scanned (ie no current in the scanning magnets). In other words, the Z -axis corresponds to the central beam path. In general, the scanning plane X-Y is also defined as a plane going through the isocenter of the scanning device.

The scanning device (20) according to the invention may be mounted on a gantry for rotation about the isocenter or it may be installed in a fixed beam line configuration or the scanning device may be integrated in any other type of system configuration.

The particle therapy assembly (100) further comprises a couch (55) for positioning the patient in an irradiation treatment position. This irradiation treatment position is the position where the target is irradiated.

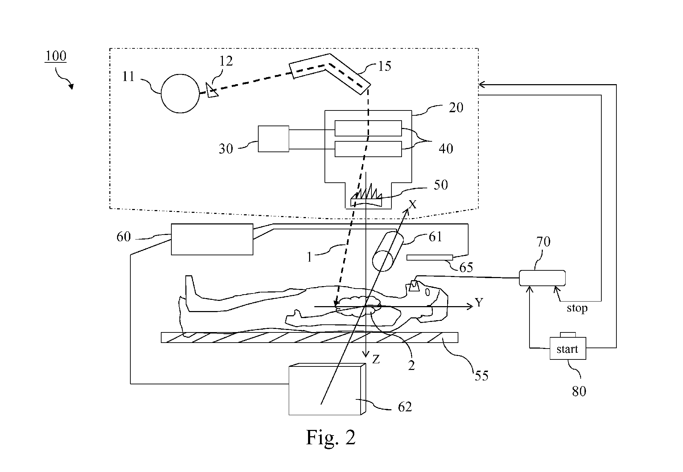

As shown schematically on Fig. 2, the particle therapy assembly according to the invention comprises means (60,61,62)for acquiring an actual position of the moving target while the patient is positioned in the treatment position. The imaging device further comprises a display (65) and displaying controls configured for displaying, in real time, on said display (65), information indicative of the actual position of the moving target and an information indicative of a prescribed target position.

The prescribed target position is defined by a medical doctor during the treatment planning phase.

The information indicative of the actual position of the moving target and the information indicative of the prescribed target position can for example be a contour of the target or it can for example be a centre point of the target.

The imaging device comprises for example a fluoroscopic imaging device. Such a fluoroscopic imaging device comprises an X-ray source (61) and an image receiver (62) that are, for example, located at 90° with respect to a central beam path through the nozzle (Z-axis shown in Fig. 2).

Alternatively, instead of using a direct imaging method such as fluoroscopic imaging, an indirect method can be used for visualizing the position of the moving target. For example an external optical tracking system can be used to track an external position of the surface of the patient and this external position can be correlated with the position of the target.

The particle therapy assembly according to the invention further comprises breath holding means (70) for holding a breath of the patient while the patient is positioned in the irradiation treatment position. Typically, the breath holding means comprises an active breath control (ABC) device known in the art. With an ABC device, the patient breathes through a mouth-piece and a valve is used to temporarily block the airflow of the patient. The purpose is to have the breath of the patient blocked during a period of 20 seconds or less. During this period the irradiation of the target is performed using the scanning device (20) according to the invention.

The particle therapy assembly (100) comprises means for actuating (80) the breath holding means to start holding the patient's breath.

The particle therapy assembly further comprises means for starting the irradiation of the target with the particle beam. This starting of the irradiation of the target is performed in relationship with the actuation of the breath holding means. This means that when the breath holding means are actuated, also the irradiation of the target with the particle beam has to be started. This can either be done separately by first actuation the breath holding means and then starting the irradiation in a second step. The time between the actuation of the breath holding means and the start of the irradiation should however be as short as possible in order to keep the breath holding period as short as possible. The start of the irradiation is preferably performed within less than one second after the actuation of the breath holding means. For this purpose, two actuating buttons can for example be installed next to each other: one for actuating the breath holding means and one for starting the irradiation immediately after actuating the breath holding means.

However, preferably, the means for actuating (80) the breath holding means is coupled to the means for starting the irradiation such that the actuation of the breath holding and the start of delivering the particle beam are performed in collaboration.

Therefore, preferably, the assembly according to the invention comprises means for synchronizing the means for starting the irradiation and the means for actuating (80) the breath holding means.

In this way, the start of the irradiation can be performed automatically without delay following the actuation of the breath holding means.

For example, the means for synchronizing comprise a common start button that triggers both the actuation of the breath-holding means and the start of the irradiation of the target. This common start button (80)is schematically represented in Fig. 2.

More preferably, the actuation of the breath holding means and the starting of the irradiation are performed simultaneously.

When the irradiation of the target is completed, the delivery of the particle beam is stopped and the breath-holding means are des-activated such that the patient can breathe normally. The synchronization means can, for example, also be configured for des-activating the breath holding means when the irradiation of the target is completed. This is illustrated schematically in Fig. 2 where the breath-holding means (70) receives a stop signal from the scanning control means (30) and/or controls of the particle beam generator when the irradiation of the target is completed.

In a preferred embodiment of the invention, the means for actuating breath holding means further comprises computing means for comparing the actual position of the moving target with the prescribed target position and means for providing a signal when the actual position of the moving target is located within a given tolerance of the prescribed target position.

Said tolerance for positioning the target is for example 30 mm, or 20 mm, or 10 mm, or 5 mm. Said tolerance is in general smaller than 50 mm, preferably smaller than 30 mm, more preferably smaller than 20 mm.

For example, the contours of the actual position of the target and the prescribed target position can be outlined on a display and when there is an overlap within the given tolerance between the two contours, a green signal is displayed.

In a preferred embodiment, the imaging device comprises means for positioning the display (65) such that the patient can visually observe the display (65) while he is in the treatment position. This means for positioning the display (65) can for example be a movable telescopic arm connected to for example the ceiling or connected to the couch (55) or connected to the scanning device (20). The display can for example be a flat panel type display. Alternatively, the means for positioning the display (65) can be a pair of glasses the patient has to put on and configured such that the display is integrated in the pair of glasses. Such glasses may also comprise two displays, one display for each eye, for providing a 3D view of the actual position of the moving target and of the prescribed target position.

In this way, having a display and the means for positioning the display such that the patient can see the screen when he is in the treatment position, the patient can actively contribute to help to position the target in the right place (i.e. the irradiation treatment position of the target) before delivering the particle beam to the target. In addition to the display positioned to be viewed by the patient, a second display in the form of a screen can, for example, be provided to the radiotherapist or other person operating the particle therapy assembly.

When the actual position of the moving target and the prescribed target position are matched within a tolerance, the breath holding means for holding the patients breath are actuated and the delivery of the particle beam is started. For performing the actuation, an actuation button can for example be used. As discussed before, this can be a single actuation button for both functions of actuating the breath holding means and starting the irradiation. This actuation button can also be a button to be selected on a computer screen.

In a first user scenario, the radio-therapist operates the actuation button and in second user scenario, the patient operates the actuation button.

In a preferred embodiment, the particle beam generator (11,12) and the scanning device (20) are configured for irradiating the target with the particle beam in 20 seconds or less.

In a more preferred embodiment, the particle beam generator (11,12) and the scanning device (20) are configured for irradiating the target with the particle beam in 10 seconds or less.

The particle therapy assembly (100) according to the invention further comprises means (50) for modulating an energy of the particle beam as function of the scanning positions (45). Under modulating the energy of the particle beam is understood that the energy of a particle beam can be varied between a minimum and a maximum value such that the penetration depth of the beam in the target is varied. In general, the modulation of the energy of the particle beam, is performed either in steps or continuous between a minimum energy value and a maximum energy value. By varying the energy of the particle beam, the position of the Bragg peak in the target is varied. By adding several Bragg peaks having different positions in the target, ie by using multiple particle beams having different energies, a so-called Spread-Out-Bragg-Peak (SOBP) is generated in the target. To each of the Bragg peaks, contributing to the SOBP, a beam intensity weight is associated. As the modulation is performed as function of the scanning position, a different Spread-Out-Bragg-Peak profile can be generated in the target for a different scanning position. A different SOBP profile is generated by applying a different energy modulation, i.e. by providing different beam intensity weights for the various energies. Depending on the beam intensity weights chosen the SOBP can be flat or it can have another non-flat shape. The minimum and maximum energy of the beam are determined based on the proximal and the distal range needed in the target for providing optimum dose conformation.

Because the energy modulation can be varied independently in the X and in the Y directions, such a means for modulating the energy permits to achieve high dose conformation to the target volume whatever the 3D shape of the target volume is.

In a preferred embodiment according to the invention, the means (50) for modulating the energy of the particle beam are installed downstream of the scanning magnets (40). In this context, "downstream" is defined with respect to the beam direction, i.e. when the means (50) for modulating the energy are installed downstream, the beam is first travelling through the scanning magnets before reaching the means (50) for modulating the energy of the particle beam. With the preferred particle therapy assembly according to the invention, due to the downstream energy modulation, the target volume can be irradiated by performing a single scan, ie the sequence of delivering the beam to the multiple scanning positions needs only to be performed one time for delivering a prescribed dose to the target. Hence, the irradiation period can be strongly reduced.

With the preferred scanning device (20) according to the invention, the irradiation period for irradiating for example a one litre target volume is 20 seconds or less.

Typical irradiation periods with current scanning devices using classical scanning techniques are of the order of one to two minutes. With those classical scanning techniques the target is divided in layers (typically 10 to 20 layers) and the irradiation of the target is performed layer per layer. Each layer corresponds to a particle energy and after each layer the energy of the beam needs to be varied upstream. The change of energy typically takes one second and this strongly increases the overall irradiation time period.

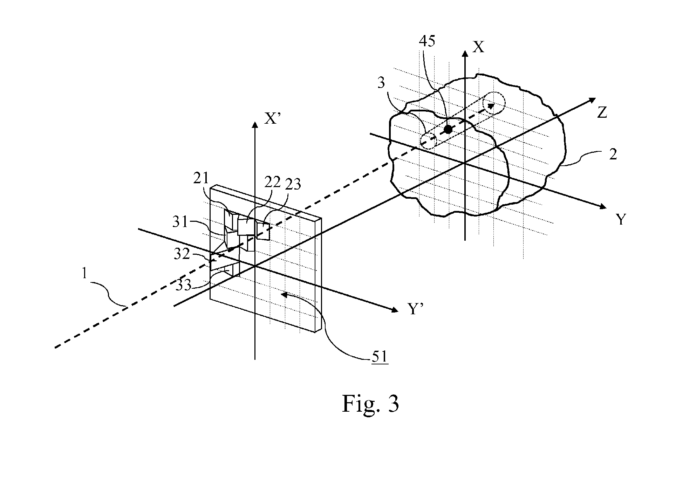

In a more preferred embodiment of the invention, the means (50) for modulating the energy comprises an energy filter (51)having a plurality of filtering elements. Each of the plurality of filtering elements is configured for modulating the energy of the particle beam and is associated to one of the scanning positions (45) of the particle beam in the X-Y plane. The energy filter is further configured and located such that when the beam is directed to a scanning position (45), the associated filtering element is being crossed by the particle beam. This is illustrated in Fig. 3, where an example of such a filter configuration is shown. This exemplary filter (51) comprises a plurality of individual filtering elements (21,22,23,.....,31,32,33,..) which are individually arranged in a transversal plane according to an X',Y' grid. For the sake of clarity, not all filtering elements are shown on this figure. In this example, both X' and Y' directions are perpendicular to each other and the filtering elements are arranged according to an orthogonal grid (shown with dotted lines), but other arrangements may of course also be used, such as non-orthogonal grids for example.

Taking into account the direction of the scanned particle beam (1) (one scanned beam (1) is indicated by a dashed line on Fig. 3), the skilled person will understand that each filtering element (23) will correspond to a particular region (3) in the target volume (2) for which a specific Spread-Out-Bragg-Peak profile is to be delivered. For a given filtering element (23), the corresponding region (3) in the target volume (2) is that part of the target volume (2) which will be irradiated with those particles having passed through the given filtering element (23).

Each of the filtering elements (21,22,23,31,32,33) comprises multiple sub-filtering elements having different material thicknesses. In this way, when the filtering element is crossed with the particle beam a distribution of particle energies is provided at the output of the filtering element.

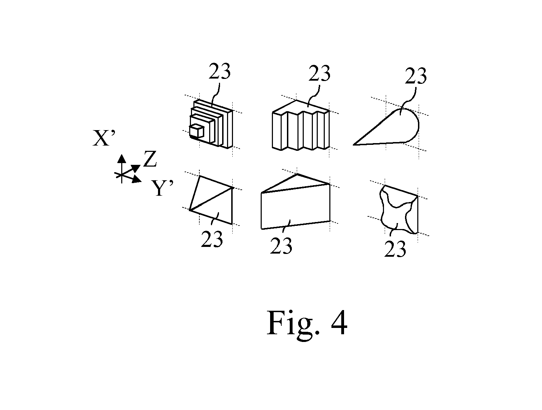

Fig. 4 shows various possible three-dimensional ("3D") shapes for a filtering element of the energy filter (50) of Fig.3. As shown on Fig.4, a filtering element (23) may for example have the shape of a 3D pyramid, or of a 3D staircase, or of a 3D cone, each of which having either stepped or continuous lateral slopes. A filtering element (23) may also have a more complex 3D shape, such as the shape shown in the bottom right part of Fig.4 for example.

For a given 3D shape of a filtering element, for example a 3D pyramid, the detailed geometrical dimensions ("the geometry") of said filtering element, such as the number of steps as well as the height and the width (frontal surface) of each step, are determined in advance in function of the desired SOBP (Spread-Out-Bragg peak) profile in the corresponding region (3) of the target volume (2). To this end, an analytical transfer function of a filtering element may be used and an optimization loop may make several iterations with this transfer function until obtaining the desired SOBP profile in the corresponding region of the target volume (2). Such methods are known from the skilled person for calculating the known ridge filters for example. Accordingly, to each region in the target volume (3) is associated a corresponding filtering element with a particular geometry. Having determined the geometries of all individual filtering elements, dedicated to a particular target volume (i.e. to a particular patient) can be built, for example by stereolithography.

Typically, the energy filter (51) has lateral dimensions (in the X'Y' plane) which substantially correspond to the frontal surface of the target volume (2). For a target volume having maximum outer dimensions of 10cm x 10cm x 10cm (according to X, Y, Z), the energy filter may for example have overall outer dimensions of about 10cm x 10cm (according to X', Y').

The number of filtering elements arranged in the transversal plane as well as their respective dimensions may be freely chosen and will depend on the required accuracy of the dose conformity.

For performing the function of shifting the energy as function of the scanning position, the energy filter (51) comprises for example a so-called range compensator which is well known to the person skilled in the art. A range compensator (sometimes called bolus) is a device specifically adapted according to the shape of the target such that the distal range of the beam is adjusted according to the shape of the target.

In a more preferred embodiment, the particle therapy assembly (100) according to the invention further comprises computing means for computing an expected irradiation period. How long a patient will be able to hold his breath will strongly vary from patient to patient. In general, it is known that a patient should be capable to hold his breath between 10 seconds and 20 seconds. Therefore, it is important to know, before launching the particle beam irradiation, how much time the irradiation will take in order to be sure that the irradiation can be performed within a single breath holding period the patient is capable of performing. In this way, the patient can be trained in advance to be able to hold his breath during a period corresponding to the expected irradiation time period. This information of expected irradiation time period and/or an additional count-down of the remaining irradiation time can preferably also be displayed on the display, visible to the patient. This will comfort the patient during the irradiation when knowing permanently the remaining time his has to hold his breath. The time period to perform the irradiation depends on the number of scan positions (45), the dose to be delivered to the target for each scanning position, the maximum beam current the particle beam generator (11,12) can deliver, the time to switch from scanning position to scanning position.

The dose to be delivered for each scan position is determined by a treatment planning system. This dose is either directly expressed in machine units or monitor units (MU) or the dose is provided in units of Gy (Gray). In the later case, a translation is first made into monitor units. The computing means will then define the beam current the particle generator should deliver. The computing means comprises a table defining the maximum beam current the particle beam generator can produce as function of the beam energy. The computing means, taking into account the maximum beam current, then calculates for each scanning position what the irradiation time is based on the dose, expressed in monitor units, to be delivered for that scanning position and a calibration factor defining the relation between monitor units and the particle beam intensity. This calibration factor will depend on the energy modulation and can hence vary from scanning position to scanning position. The calibration factor can be experimentally determined and stored in calibration tables. The computing means comprises these calibration tables defining this relation between monitor units and particle beam intensity. By summing the irradiation times of all scanning positions plus taking into account the time for switching the particle beam to all scanning positions, the expected irradiation time period is computed by the computing means.

The expected irradiation period is preferably displayed on the display (65).

According to a second aspect of the invention a method is provided for positioning a moving target of a patient. This method comprises the steps of
a. positioning the patient in a treatment position for particle radiation therapy;
b. providing breath holding means (70) for holding the patient's breath;
c. providing an imaging device for acquiring and visualizing an actual position of the moving target while the patient is in the treatment position and the imaging device comprises a display and displaying controls configured for displaying, in real time, an information indicative of the actual position of the moving target and an information indicative of a prescribed target position ;
d. positioning the display (65) such that the patient can visually observe the display (65) while the patient is located in the treatment position;
e. displaying, in real time, the information indicative of the actual position of the moving target together with the information indicative of the prescribed target position on said display (65);
f. having the patient managing his respiratory such that the actual position of the moving target matches the prescribed target position within a tolerance, said managing being performed based on said information indicative of the actual position of the moving target and said information indicative of the prescribed target position;
g. actuating the breath holding means to start holding the patient's breath when the actual position of the moving target matches the prescribed target position within said tolerance.

Said tolerance can vary from case to case and is generally defined by a medical doctor. It is generally smaller than 30 mm. Typically, the tolerance has a value of 30 mm, or 20 mm, or 10 mm, or 5 mm, or 2 mm.

By actively controlling his breath, the patient can vary the inflation level of his lungs to adjust or bring the actual position of the target close to the prescribed target position. To help to compare the actual position and the prescribed position, the contours of the target in the actual position and the contours of the target in the prescribed position can be outlined on the display. As an example, a green signal, indicating when the actual and prescribed positions are matching within the tolerance, is displayed on the display.

With this step of having the patient actively helping to bring the target in the prescribed position, the reproducibility of the position of the target and the organs at risk can be strongly improved.

In one user scenario, the actuation of the breath holding means is performed by the patient himself while being located in the treatment position. When the actual position corresponds, within some tolerance, to the prescribed position, the patient can push, for example, an actuation button to actuate the breath holding means.

In an alternative user scenario, the patient can adjust his breath using the visualization on the display of the actual position of the target as discussed before, but, the actuation of the breath holding means and the starting of the irradiation is performed by a radio-therapist or other person located in the treatment control room who is observing the same visualization of the actual and prescribed target position.

As another example, instead of using an actuation button, computer controls can perform a comparison between the actual position of the target and the prescribed position of the target. When, within a given tolerance, both positions are matching the computer controls can then automatically actuate the breath holdings means.

The present invention has been described in terms of specific embodiments, which are illustrative of the invention and not to be construed as limiting. More generally, it will be appreciated by persons skilled in the art that the present invention is not limited by what has been particularly shown and/or described hereinabove. The invention resides in each and every novel characteristic feature and each and every combination of characteristic features.

Reference numerals in the claims do not limit their protective scope.

Use of the verbs "to comprise", "to include", "to be composed of", or any other variant, as well as their respective conjugations, does not exclude the presence of elements other than those stated.

Use of the article "a", "an" or "the" preceding an element does not exclude the presence of a plurality of such elements.

## Claims

1. A particle therapy assembly (100) for irradiating a moving target (2) in a patient with a particle beam (1), said particle therapy assembly (100) comprising :
● a particle beam generator (11,12) for generating the particle beam (1);
● a scanning device (20) for scanning said target (2) with said particle beam, said scanning device (20) comprising :
● one or more scanning magnets (40) for scanning the particle beam over an X-Y scanning plane;
o scanning control means (30) configured for scanning the particle beam by sequentially moving the particle beam to multiple scanning positions (45) situated in said X-Y scanning plane;
● a patient couch (55) for positioning said patient in an irradiation treatment position;
● an imaging device (60,61,62,65) configured for acquiring an actual position of said moving target while the patient is in said irradiation treatment position, said imaging device comprising a display (65) and displaying controls configured for displaying, in real time, on said display (65), information indicative of the actual position of said moving target and information indicative of a prescribed target position;
● breath holding means (70) for holding a breath of said patient while said patient is positioned in said irradiation treatment position;
● means for actuating (80) said breath holding means (70) to start holding the patient's breath; and
● means for starting an irradiation of the target with the particle beam.

2. A particle therapy assembly (100) according to claim 1, **characterized in that** said particle therapy assembly (100)further comprises means for positioning said display (65) such that said patient can visually observe said display (65) while said patient is in said irradiation treatment position.

3. A particle therapy assembly (100) according to any of previous claims **characterized in that** said display (65)is in a display position which is such that said patient can visually observe said information indicative of the actual position of said moving target and said information indicative of a prescribed target position while said patient is in said irradiation treatment position.

4. A particle therapy assembly (100) according to any of previous claims, further comprising :
● computing means for comparing in real time said actual position of said moving target with said prescribed target position, and
● means for providing a signal when said actual position of said moving target matches said prescribed target position within a tolerance.

5. A particle therapy assembly (100) according to any of previous claims, further comprising means for synchronizing said means for starting the irradiation and said means for actuating (80) the breath holding means.

6. A particle therapy assembly (100) according to claim 5 wherein said means for starting the irradiation and said means for actuating (80) the breath holding means are configured such that, in operation, said starting the irradiation is performed at the same time as said actuating the breath holding means.

7. A particle therapy assembly (100) according to any of previous claims wherein said imaging device comprises a fluoroscopic imaging device.

8. A particle therapy assembly (100) according to any of previous claims wherein said means for actuating (80) the breath holding means comprises an actuation button operable by a human operator.

9. A particle therapy assembly (100) according to any of previous claims **characterized in that** it further comprises computing means for computing an expected irradiation time period for irradiating said target.

10. A particle therapy assembly (100) according to any of previous claims wherein said breath holding means comprises an active breath control (ABC) device.

11. A particle therapy assembly (100) according to any of previous claims, further comprising means (50) for modulating an energy of the particle beam as function of said scanning positions (45, said means (50) for modulating the energy being located downstream of all of said one or more scanning magnets (40).

12. A particle therapy assembly (100) according to claim 11 wherein said means (50) for modulating the energy comprises an energy filter (51), said energy filter (51) having a plurality of filtering elements (21,22,23,31,32,33), each of said plurality of filtering elements being configured for modulating the energy of the particle beam and being associated to one of said scanning positions (45) of the particle beam in the X-Y scanning plane.

13. A particle therapy assembly (100) according to claim 12, **characterized in that** each of said filtering elements (21,22,23,31,32,33)comprises multiple sub-filtering elements having different material thicknesses.

14. A particle therapy assembly (100) according to any of previous claims, **characterized in that** the particle beam generator (11,12) and the scanning device (20) are configured for irradiating the target with the particle beam in 20 seconds or less.

15. A method for positioning a moving target (2) of a patient, said method comprising the steps of
a. positioning said patient in an irradiation treatment position;
b. providing breath holding means (70) for holding the patient's breath;
c. providing an imaging device (60,61,62,65) configured for acquiring an actual position of said moving target while the patient is in said irradiation treatment position, said imaging device comprising a display (65) and displaying controls configured for displaying, in real time, on said display (65), information indicative of the actual position of said moving target and information indicative of a prescribed target position ;
d. positioning said display (65) such that the patient can visually observe the display (65) while the patient is located in said irradiation treatment position;
e. displaying, in real time, on said display (65), the information indicative of the actual position of said moving target together with the information indicative of the prescribed target position ;
f. having the patient managing his respiratory such that the actual position of said moving target matches the prescribed target position within a tolerance, said managing being performed based on said information indicative of the actual position of the moving target and said information indicative of the prescribed target position;
g. actuating the breath holding means (70) to start holding the patient's breath when said actual position of the moving target matches said prescribed target position within said tolerance.

16. A method according to claim 15 wherein said actuation of the breath holding means (70) is performed by the patient while positioned in said treatment position.

17. A method according to claim 15 wherein said actuation of the breath holding means (70) is performed by a person other than said patient.
